(19) Europäisches Patentamt
European Patent Office
Office européen des brevets

(11) Publication number: **0 052 909**
**B1**

(12) **EUROPEAN PATENT SPECIFICATION**

(45) Date of publication of patent specification: **10.04.85**

(51) Int. Cl.$^4$: **C 07 C 153/11,** A 61 K 31/265
// C07C57/72 ,(A61K31/265, 31:71),(A61K31/265, 31:195)

(21) Application number: **81201308.4**

(22) Date of filing: **25.11.81**

(54) **Derivative of alfa-mercaptopropionilglicine having therapeutical activity, process for its preparation and related pharmaceutical compositions.**

(30) Priority: **26.11.80 IT 2623280**

(43) Date of publication of application:
**02.06.82 Bulletin 82/22**

(45) Publication of the grant of the patent:
**10.04.85 Bulletin 85/15**

(84) Designated Contracting States:
**BE DE FR GB**

(56) References cited:
**DE-A-2 917 790**
**GB-A-2 054 586**
**US-A-3 246 025**

(73) Proprietor: **Real S.a.s. di Alberto Reiner & C.**
**Via Cattaneo, 1**
**I-22063 Cantù (Como) (IT)**

(72) Inventor: **Reiner, Alberto**
**Via Mentana, 23**
**Como (IT)**

(74) Representative: **Dragotti, Gianfranco**
**SAIC BREVETTI S.a.s. di Ing. G. Dragotti & C. Via Spontini, 1**
**I-20131 Milano (IT)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 052 909 B1**

**Description**

The present invention relates to a thioester of α-mercapto propionylglicine with p-isobutylphenylpropionic acid and having the formula:

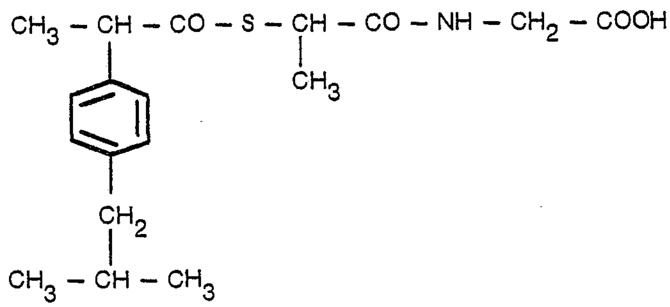

having interesting anti-inflammatory and mucolytic properties, as well as to its non toxic and pharmacologically acceptable salts. The anti-inflammatory activity of the p-isobutylphenylpropionic acid is known, and there are also known its disadvantages from the therapeutical and use point of view.

The compound of the invention, as such or converted to a salt with inorganic bases containing alkaline or earth-alkaline ions, or with basic aminoacids, such as arginine and lysine, or with nuclei of basic antibiotics, such as erythromycin and propionylerythromycin, shows interesting properties, since it is simultaneously endowed with anti-inflammatory and mucolytic activity.

The present invention relates also to the process for the preparation of the subject compound, which is characterized in that the chloride of p-isobutylphenylpropionic acid and the α-mercaptopropionylglycine are reacted in a medium having alkaline pH comprising a polar solvent, preferably water or mixtures of water and dioxane, at low temperature, giving place to a low melting thioester which, at room temperature, is an odorless pale yellow oil. The reaction product, as such is devoid of impurities and is utilized in the salt forming reaction with the several bases above referred to, in order to obtain the corresponding salts, which are stable, water soluble and easy to be used in the pharmaceutical field. The following example illustrates, in non limiting sense, the process for the preparation of the derivative of the invention.

Example

a) preparation of the chloride of p-isobutylphenylpropionic acid

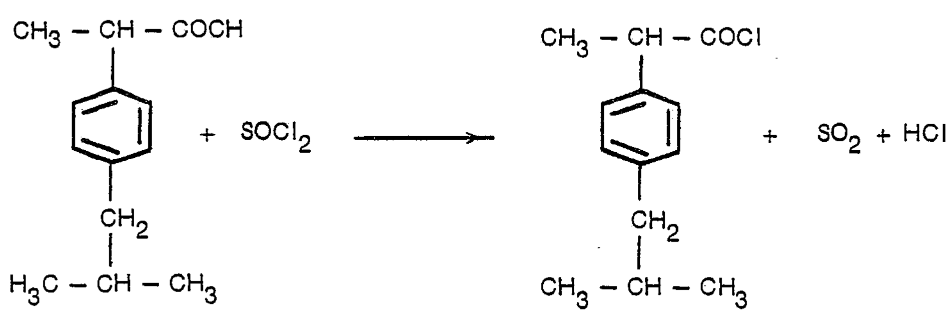

A flask is charged with 600 mls of chloroform and 250 mls of $SOCl_2$ and this solution is slowly supplemented with 500 g of p-isobutylphenylpropionic acid. The latter is dissolved by lowering the temperature to value near 0°C. Then a heating to 20—25°C is carried out and at that point $SO_2$ and HCl are developed. The heating is slowly continued up to 60—63°C, the reaction being monitored on the basis of the gas development and until the latter ceases. The reaction mixture is concentrated under vacuum to eliminate the solvent and the unreacted thionyl chloride, and an oil is obtained which is used as such for the subsequent reaction. There are obtained 540 g of the chloride of the p-isobutylphenylpropionic acid

b) preparation of p-isobutylphenylpropionyl α-mercapatopropionylglycine

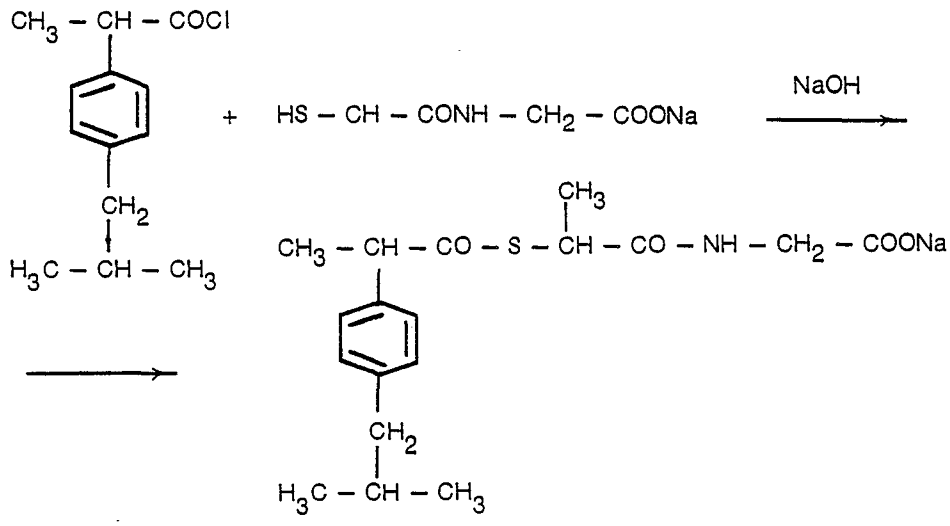


in a flask having a stirrer, 10 g of the sodium salt of α-mercaptopropionylglycine and then 80 mls of water are charged. A cooling is carried out so that the internal temperature is about 0°C. At this point the simultaneous addition, through two drop adding funnels, of 2,2 g of NaOH dissolved in 15 mls of water and 12,5 g of the previously obtained chloride is started. The addition takes place by maintaining the temperature always at about 0°C and for a time of about 3 hours. After that time the temperature is let to slowly rise up to the room temperature, a control sample being taken. The pH of the solution is neutral. There are added 80 mls of water and the mixture is made acidic with concentrated HCl until pH=1. The reaction mixture is extracted with methyl isobutylketone and separated from the aqueous phase.
The methylisobutylketone solution is dried and is concentrated to dryness.

c) Preparation of the (L) lysine salt of p-isobutylphenylpropionoylthio-α-mercaptopropionylglycine

1 mole of p-isobutylphenylpropionoylthio-α-mercaptopropionylglycine acid dissolved in alcohol is reacted with a water solution of (L)-lysine base at a concentration of 50% and there is obtained, with a yield of 72%, a pure crystalline product formed by the (L)-lysine salt of p-isobutylphenylpropionoylthio-α-mercapto propionylglycine, having melting point of 152—153°C. By proceeding in a like manner, but using (DL)-lysine base, the salt obtained has a melting point of 125—127°C.

d) preparation of the propionylerythromycin salt of p-isobutylphenylpropionoylthio-α-mercapto-propionylglycine

The salt forming reaction takes place in an aqueous solution of 2% methylisobutylketone or in alcohol with a yield of 85% of the theoretical value, between one mole of propionylerythromycin and one mole of p-isobutylphenylpropionoylthio-α-mercaptopropionylglycine.

The product is in form of straw coloured crystals having melting point of 71—72°C. By proceeding in a like manner, but using one mole of erythromycin base, the related salt is obtained having a melting point of 65—67°C.

The inorganic salts of sodium and potassium are highly soluble in water, whereas the calcium and magnesium salts are insoluble. They can be obtained from p-isobutylphenylpropionoylthio-α-mercaptopropionylglycine acid and preferably by reaction with the respective alkaline or earth-alkaline carbonates or bicarbonates.

All the salts are partially or totally soluble in alcohols.

The derivative according to the invention has been the subject of pharmacological tests aiming to determine both the toxicity and the therapeutical effects.

1) Acute toxicity

Sprague-Dawley albine rats having an average weight of 120 ± 5 g and of both sexes were used.

For each compound 40 rats were treated. The test compound as a suspension in 5% arabic gum was administered through a gastric probe.

The results were evaluated ten days after the administration.

The data reported in the table demonstrate that it was not possible to obtain a value of the LD 50, since also a dosage of 900 mg/Kg did not cause any lethal effect, as it is seen from the enclosed table 1.

TABLE 1

Acute toxicity by oral route in the rat

| Treatment | dead/living | % mortality |
|---|---|---|
| 150 | 0/8 | 0 |
| 300 | 0/8 | 0 |
| 450 | 0/8 | 0 |
| 600 | 0/8 | 0 |
| 900 | 0/8 | 0 |
| $LD_{50}$ | 900 mg/kg | |

2) Anti-inflammatory activity

The tests used for the evaluation of the anti-inflammatory activity were respectively that of the kaolin and that of the egg white.

a) Anti-inflammatory activity in the kaolin test. For each compound 16 rats of male sex, of the Sprague-Dawley strain and of 200—250 g of body weight, were used. The rats were divided into four groups each comprising four rats. All the animals were injected with a 10% suspension of kaolin. The first group was used as the control, whereas the other three groups were administered with doses respectively of 50,75 and 100 mg/Kg. The measurements of the diameters of the articulation were effected one hour after the kaolin injection and then every 24 hours.

The obtained data are reported in the table 2. The inoculation of kaolin caused an increase of the tibio-tarsal articulation which has been partially limited by the treatment with the test compound.

TABLE 2

Kaolin test

| Doses | Anti-Inflammatory Activity % |
|---|---|
| Controls (kaolin) | – |
| 50 mg/kg | – |
| 75 mg/kg | 10 |
| 100 mg/kg | 26 |

b) Egg white test.

For each compound 16 male rats, of the Sprague-Dawley strain and weighing 300 g, were used. The animals were divided in four groups each comprising four rats. The first group served as a control, whereas the other three groups were orally administered, with a 30 minute interval, with two doses of 50,75 an 100 mg/Kg of the test compound.

The last administration was immediately followed by the inoculation of 0,1 ml of egg white in the rear paw of all the animals, including the controls. The measurement of the diameters of the articulations were effected 3 and 4 hours later.

The data obtained in the present test are reported in the table 3.

TABLE 3

Egg white test

| Doses | Anti-Inflammatory Activity % |
|---|---|
| Controls | – |
| 50 mg/kg | – |
| 75 mg/kg | – |
| 100 mg/kg | 26 |

The subject compound was also tested with respect to a possible teratogenic action, by administering to female rats a dose of 50 mg/Kg from the beginning up to the 19-20 day of gestation. It has been found that no significant difference exists as regards the reabsorption number and the foeti in the treated rats with respect to the controls. Also the weight of the foeti did not revealed whatsoever anomaly.

The thioester of α-mercaptopropionylglycine with p-isobutylphenylpropionic acid according to the present invention and the aforesaid salts are formulated by the known techniques, excipients, solvents, fillers, etc, giving place to pharmaceutical compositions in form of tablets, suppositories, injectable solutions, suspensions, syrups, emulsions, creams and aerosols.

In fact the stability and solubility of the subject compound and of its salts allow for such a complete range of uses.

**Claims**

1. Thioester of α-mercaptopropionylglycine with p-isobutylphenylpropionic acid having the formula:

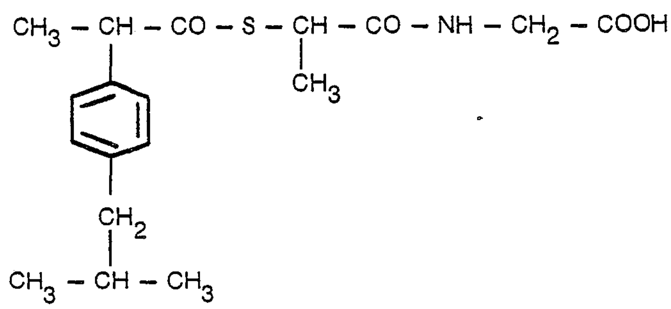

and the non toxic and pharmaceutically acceptable salts thereof with organic and inorganic bases.

2. Thioester according to claim 1, characterized in that said salts are formed with inorganic bases containing alkaline and earth-alkaline ions.

3. Thioester according to claim 1, characterized in that said salts are formed with basic aminoacids.

4. Thioester according to claim 3, characterized in that said basic aminoacids are arginine and lysine.

5. Thioester according to claim 1, characterized in that said salts are formed with basic antibiotics.

6. Thioester according to claim 5, characterized in that said basic antibiotics are erythromycin and propionylerythromycin.

7. A process for the preparation of the thioester of α-mecaptopropionylglycine with p-isobutylphenylpropionic acid, characterized in that the chloride of the p-isobutylpropionic acid is reacted with α-mercaptopropionylglycine in a polar medium at an alkaline pH and at low temperature.

8. A process according to claim 7, characterized in that said polar medium comprises water or mixtures of water and dioxane and the reaction temperature is of about 0°C.

9. Pharmaceutical compositions having anti-inflammatory and mucolytic activity, characterized by containing together with the common excipients and/or solvents, an effective amount of the thioester of α-mercaptopropionylglycine with p-isobutylphenylpropionic acid or a non toxic and pharmacologically acceptable salt thereof according to claims 1 to 6.

10. Pharmaceutical composition according to claim 9, in form of tablets, syrups and suspensions for the oral administration.

11. Pharmaceutical composition according to claim 9, in form of solution for the parenteral administration.

12. Pharmaceutical composition according to claim 9, in form of suppositories for the rectal administration.

13. Pharmaceutical composition according to claim 9, in form of cream, ointment, aerosol and the like for topical administration.

**Patentansprüche**

1. Thioester von alpha-Mercaptopropionylglycin mit p-Isobutylphenylpropionsäure der Formel:

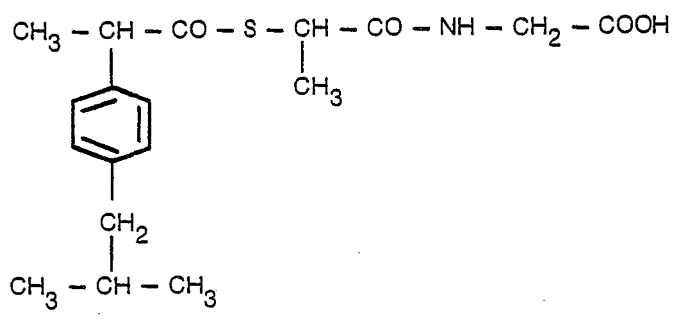

und die nicht toxischen und pharmazeutisch annahmbaren Salze davon mit organischen und anorganischen Basen.

2. Thioester nach Anspruch 1, dadurch gekennzeichnet, daß die genannten Salze mit alkalische und erdalkalische Jonen enthaltenden anorganischen Basen gebildet sind.

3. Thioester nach Anspruch 1, dadurch gekennzeichnet, daß die genannten Salze mit basischen Aminosäuren gebildet sind.

4. Thioester nach Anspruch 3, dadurch gekennzeichnet, daß die genannten basischen Aminosäuren Arginin und Lysin sind.

5. Thioester nach Anspruch 1, dadurch gekennzeichnet, daß die genannten Salze mit basischen Antibiotika gebildet sind.

6. Thioester nach Anspruch 5, dadurch gekennzeichnet, daß die genannten basischen Antibiotika Erythromycin und Propionylerythromycin sind.

7. Ein Verfahren zur Herstellung des Thioesters von alpha-Mercaptopropionylglycin mit p-Isobutylphenylpropionsäure, dadurch gekennzeichnet, daß das Chlorid der p-Isobutylphenylpropionsäure in einem polaren Medium bei einem alkalischen pH und bei einer niedrigen Temperatur mit alpha-Mercaptopropionylglycin umgesetzt wird.

8. Ein Verfahren nach Anspruch 7, dadurch gekennzeichnet, daß das genannte polare Medium Wasser oder Wasser-Dioxan-gemische umfasst und daß die Reaktionstemperatur umgefähr bei 0°C liegt.

9. Pharmazeutischen Zusammensetzung mit anti-inflammatorischer und mucolytischer Wirksamkeit, dadurch gekennzeichnet, daß sie zusammen mit den üblichen Bindemitteln und/oder Lösungsmitteln eine effektive Menge des Thioesters von alpha-Mercaptopropionylglycin mit p-Isobutylphenylpropionsäure oder ein nicht toxisches und pharmakologisch annahmbares Salz davon nach den Ansprüchen 1 bis 6 enthält.

10. Pharmazeutischen Zusammensetzung nach Anspruch 9, in Form von Tabletten, Syrups und Aufschlämmungen zur oralen Verabreichung.

11. Pharmazeutischen Zusammensetzung nach Anspruch 9, in Form einer Lösung zur parenteralen Verabreichung.

12. Pharmazeutischen Zusammensetzung nach Anspruch 9, in Form von Suppositorien zur rectalen Verabreichung.

13. Pharmazeutische Zusammensetzung nach Anspruch 9, in Form einer Creme, Salbe, Aerosol und dlg zur topicalen Verabreichung.

**Revendications**

1. Thioester de l'α-mercaptopropionylglycine avec l'acide p-isobutylphenylpropionique ayant la formule

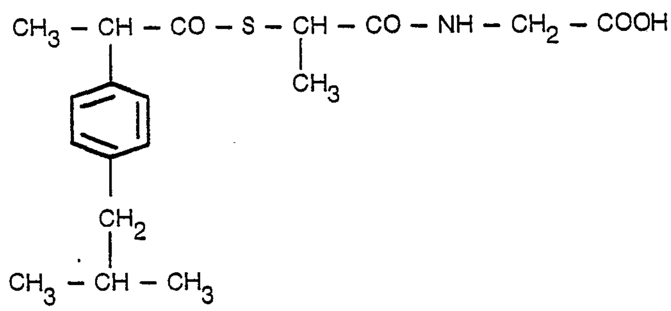

et ses sels non toxiques et pharmaceutiquement acceptables avec des bases organiques et inorganiques.

2. Thioester selon la revendication 1, caractérisé en ce que les dits sels sont formés avec des bases inorganiques contenant d'ions alcalins et alcalin-terreux.

3. Thioester selon la revendication 1, caractérisé en ce que les dits sels sont formés avec des aminoacides basiques.

4. Thioester selon la revendication 3, caractérisé en ce que les dits aminoacides basiques sont arginine et lysine.

5. Thioester selon la revendication 1, caractérisé en ce que les dits sels sont formés avec des antibiotiques basiques.

6. Thioester selon la revendication 5, caractérisé en ce que les dits antibiotiques basiques sont érythromycine et propionylérythromycine.

7. Un procédé pour la préparation du thioester d'alpha-mercaptopropionylglycine avec l'acide p-isobutylphenylpropioniques, caractérisé en ce qu'on fait reagir le chlorure de l'acide p-isobutylphenylpropionique avec l'alpha-mercaptopropionylglycine dans un milieu polaire à un pH alcalin et à une faible température.

8. Un procédé selon la revendication 7, caractérisé en ce que le dit milieu polaire comprend l'eau ou des melanges de l'eau avec dioxane et la température de reaction est d'à peux prés 0°C.

9. Composition pharmaceutique ayant activité anti-inflammatoire et mycolitique, caractérisé en ce qu'elle contient, avec les excipients et/ou solvants communs, une quantité efficace du thioester de l'α-mercaptopropionylglycine avec l'acide p-isobutylphenylpropionique ou d'un de ses sels non toxiques et pharmacologiquement acceptables selon les revendications 1 à 6.

10. Compositon pharmaceutique selon la revendication 9, sous forme de comprimés, sirops, et suspensions pour l'administration orale.

11. Composition pharmaceutique selon la revendication 9, sous forme de solution pour l'administration parentérale.

12. Composition pharmaceutique selon la revendication 9, sous forme de suppositoires pour l'administration rectale.

13. Composition pharmaceutique selon la revendication 9, sous forme de crème, onguent, aerosol et semblables pour l'administration topique.